# EUROPEAN PATENT APPLICATION

(11) **EP 1 502 959 A1**
(43) Date of publication of application: **02.02.2005**
(21) Application number: 03017134.2
(22) Date of filing: 29.07.2003
(51) Int. Cl.: C12Q 1/68

(54) **A procedure for the creation and use of DNA-recognition element libraries in vivo in relation to gene expression**

(71) Applicant: Hans-Knöll-Institut für Naturstoff-Forschung e.v., 07745 Jena (DE)
(72) Inventor: Saluz, Hans Peter, Prof. Dr., 07646 Oberbodnitz (DE); Tretyakov, Alexandre, Dr., Leningrad Area, 188300 Gatchina City (RU); Wu, Zhihao, Dr., Anhui Normal University, 241000 Wuhu, AnHui Province (CN)
(74) Representative: Kelch, Ekkehard

(57) **Abstract**

An universal technique to identify genomic DNA-binding elements in vivo that interact with known and well characterized biomolecules like transcription factors, on the entire genomic DNA in any living organisms, tissues or cells (prokaryotes and/or eukaryotes). This technique allows the determination of the associated genes in silico. The DNA sequences are determined containing both, the recognition elements of the biomolecules on the DNA and parts of the corresponding genes that can be used for the expression analysis of the target genes, or are modulated by the complex formation of, for example, transcription factors. This technique enables the simultaneous identification of DNA-recognition elements and expression analysis of target genes and an assessment of its importance for gene expression.

This technique is used to develop transcription-factors and other DNA-interacting biomolecules-specific recognition element libraries including flanking DNA sequences, e.g. coding sequences.

## Description

The procedure allows to capture protein/DNA-complexes as they occur *in vivo,* by crosslinking the proteins to their target sites following, for example, nanosecond-high energy UV-pulse irradiation of living tissue or cells in culture. The DNA fragments associated with a specific protein are selected by immunoprecipitation and subsequently cloned to enable their identification by sequencing. The resulting recognition element DNA (reDNA) library represents a compilation of *in vivo* binding sites plus flanking sequences and can be constructed for any well characterized DNA-binding protein. Moreover, the sequence information obtained from regions surrounding the binding sites provides a valuable tool in the search for the corresponding gene. What is more, this procedure enables the simultaneous identification of DNA-recognition elements and expression analysis of target genes and an assessment of its importance for gene expression.
For this, the DNA sequences are selected containing both, the recognition elements of the biomolecules on the DNA and parts of the corresponding genes that can be used for the expression analysis of the target genes, or are modulated by the complex formation of, for example, transcription factors.

Currently, there are many different techniques available to identify transcription factor binding sites *in vitro.* These methods known as CASTing, SELEX, SAAB, TDA or MuST consist either of synthesizing randomized oligonucleotides with a defined flanking sequence as PCR primer or of genomic DNA fragments ligated to defined linkers for the following amplifications. This randomized DNA molecule population is mixed with a purified DNA-binding protein or with a nuclear extract and the specific protein/DNA-complexes are isolated by one of the various procedures, such as immunoprecipitation or filter binding.
Although protein/DNA-interaction studies *in vitro* provide valuable information, it is quite obvious that the information obtained by this means does not necessarily reflect the actual situation *in vivo.* For instance, studies on the binding of proteins to DNA *in vitro* do not take the role of DNA-chromatin structure into account which modulates gene expression through the different levels of organization: nucleosomes and their positioning and the higher order structure of chromatin. The only way to overcome these shortcomings is to work in parallel with living cells. For the purpose of understanding the term "in vivo" is described as follows: All living systems, e.g. bacteria, drosophila etc., as intact living systems or intact organs or tissue or cells or nuclei isolated from organs and other functional entities.

This invention enables the universal detection of genomic DNA binding elements to develop transcription factors and other DNA-interacting biomulecule-specific libraries. It enables, for example the identification of genomic targets of drugs, chemicals, toxins, pathogens and environmental factors and can be used for elucidating the molecular perturbations and the relative contribution of these events for essentially all human and animal or plant and other organism-specific diseases at the genome level.

### Figure :Illustrates the scheme of the principal procedure of this invention.

The first aspect of this invention concerns the technology for the construction of the recognition element libraries. The technique is based on the creation of a DNA recognition element library by isolation of specific nucleoprotein complexes (1) that have been fixed in living cells (2) by protein/DNA cross linking (3), for example by UV-pulse laser irradiation. The fixation conditions for nucleoprotein complexes are chosen in such way that on average only one biomolecule is bound per DNA-restriction fragment leading to appropriate DNA fragments of, for example, one nucleoprotein complex per 1000 bp length (1). Upon an appropriate digestion (4) with restriction endonucleases, such as Bam HI, linker ligation (5) and an exonuclease digestion (6) with, for example Strandase, the protein complexed to the DNA is digested with a protease (7), such as Proteinase K. Following a primed extension reaction (8), to obtain double stranded, blunt-ended DNA fragments, and a second linker ligation step (9) to the said blunt-DNA ends, a ligation-mediated polymerase chain reaction (LMPCR) is performed (10) to amplify the various specific DNA-recognition elements including their flanking sequences. The LMPCR products are cloned (11) for the purpose of DNA mass production. Subsequently, DNA-sequence analysis (12) is carried out for the creation of the DNA-recognition libraries of former nucleoprotein and/or biomolecule complexes. This invention provides unique information on protein and/or biomolecule/DNA-interaction in conjunction with gene expression data. The individual recognition elements can now be, for example, immobilized and precisely positioned on the surface of solid bodies, such as chip surfaces (13), for further analysis to obtain library information, as said herein, e.g., gene determination, footprint- and differential gene expression patterns. The sequence analysis of the various cloned DNA fragments allows the assignment to the corresponding target genes and ascertains the biomolecular/DNA-recognition elements. This information can be obtained on the basis of fixation of nucleoprotein and or biomolecular-DNA complexes which contain double stranded DNA recognition elements and subsequent exonuclease treatment as described above which results in single stranded and uncomplexed DNA. It is important to realize that the exonuclease treatment leads to single-stranded DNA outside of the nucleoprotein complex. Thus, the DNA-footprint information for individual biomolecular/DNA complexes is based on double stranded DNA within the occupied biomolecular/DNA complexes. This information is obtained by an *in silico* determination of the complementary DNA sequence elements, i.e. the recognition elements and some additional nucleotides according to the chosen exonucleases. The expression patterns are obtained upon hybridization (15) of immobilized DNA fragments, e.g. the individual molecules of the library, with labeled mRNA or labeled cDNA thereof.

The second aspect of this invention concerns the use of recognition element libraries for the identification of genomic targets of, for example, viral proteins that interact with human regulatory DNA-elements and thus causing severe changes in the corresponding gene expression.
The third aspect of this invention concerns diagnostic systems involving protein/DNA-interactions *in vivo* in dependence of the correlated gene expression.

### Example 1: The production of a recognition element library based on the transcription factor cJun.

Thin slices of rat *cerebellum* are prepared in oxygenated Hank's saline (Gibco) and irradiated in Petri dishes with a short pulse of UV-laser-light (10 ns, 10 to 15 mJ/cm²)[Wittig, B., Wölfl, S., Dorbic, T., Vahrson, W. & Rich, A. (1992) *EMBO* J: 11, 4653-4663; Wölfl, S., Martinez, C., Rich, A. & Majzoub, J.A. (1996) Proc. Natl. Acad. Sci. USA 93, 3664-3668]. Following irradiation, the tissue is disrupted and nuclei isolated. The nuclei are resuspended in 1 ml of 10 mM Tris-HCl, pH 7.9, 150 mM NaCl, 10 mM MgCl₂, 1 mM PMSF and incubated with 50 units of BamHI at 37° C. Then nuclei are lysed, and overlaid on a CsCl gradient as described elsewhere [Gilmour, D.S., Rougvie, A.E. & Lis, J.T. (1991) in *Functional Organization of the Nucleus.* Ed. Hamkalo. B.A. und Elgin, C.R. (Academic Press, Inc.)]. Upon ultracentrifugation 500-µl fractions are collected and their absorbance at 260 and 280 nm is measured. In addition a slot blot experiment is performed to identify the cJun/DNA-complexes. The positive slot-blot fractions are collected, dialyzed twice against a vast excess of 0.2% Sarcosyl, 50 mM Tris-Cl (pH 8), 2 mM NaEDTA (pH 8) for 24 hours at 4°C and once against 2.5% glycerol, 25 mM Tris-Cl (pH 8), 2 mM NaEDTA (pH 8), restriction digested with Sau 3AI, subjected to a specific immunoprecipitation using 1 µg of rabbit-anti-cJun antibodies in 1% Triton X-100, 0.1% NaDeoxycholate, 150 mM NaCl, 1 mM PMSF overnight at 4°C. The immunoprecipitation is completed by adding protein-G Sepharose to bind the antibodies and the specific protein/DNA complexes bound the Antibodies-Sepharose complexes are precipitated by centrifugation in a clinical centrifuge. The precipitate is washed twice in PBS at 4 °C. The specific purified complexes are subjected to a first linker-ligation reaction (5'-phosphorylated linker, 3'-end blocked by NH₂-C₆) and digested with approx. 10 units of "Strandase" for 2 hours at 37°C.
The sample is dissolved in 300 µl of 10 mM Tris-HCl, pH 7.8, 5 mM NaEDTA, 0.5 % (w/v) SDS and digested with 0.1 mg/ml Proteinase K at 37° C overnight. After phenol extraction and ethanol precipitation, primed extension using Pfu polymerase (2.5 U/50 µl reaction volume; 10 min at 72°C) and a primer complementary to the lower strand of the first linker (see above) is performed.
The now double-stranded blunt-ended DNA is ligated to a second linker (unmodified), the upper 5'-end of which is overlapping. The DNA is subjected to ligation-mediated PCR as follows. The purified selected DNA is taken up in 10 µl water. One tenth of the reaction mixture (1 µl) is exponentially amplified (total volume: 50 µl; conditions: 25 pmol of primer 1; 10 mM Tris-HCl, pH 8.3, 500 mM KCl, 1.5 mM MgCl2, 150 mM each of dATP, dCTP, dGTPand dTTP, 2.5 units of Pfu Polymerase (Stratagene); 25 cycles; cycle conditions: 1 min at 94° C, 0.5 min at 52° C, 1.5 min at 72 °C). To increase the amount of amplified DNA, an additional amplification step (30 cycles) is performed with one thousand dilution of the initial mixture under the above conditions. The unextended primers are removed Qiagen PCR-Purification kit). Upon phosphorylation of the purified PCR-products (1mM ATP, 1 µl T4 kinase [5 units; New England Biolabs]), the phosphorylated amplified fragments are cloned into the EcoRV site of pBluescript II KS (L).
Sequence analysis, immobilization, labeling, hybridization and detection are performed according to various manufacturers recommendations or standard protocols, respectively.

### Example 2: Identification of novel cJun target in rat-brain tissue:

To demonstrate the power of gDNA-libraries we elucidated *in vivo* the interaction sites of the proto-oncogene-protein c-Jun, a member of the AP-1 transcription factor family, in rat *cerebellum,* where expression of c-Jun is high. Neuronal excitation elicits rapid transcriptional activation of several immediate-early genes, such as c-Jun. Many immediate-early genes encode transcription factors that control expression of downstream genes the products of which seem to bring about long-term plasticity changes. The central nervous system continuously receives information of the exterior, and a central role has been proposed for the early responsive genes as "third messengers" in the cytoplasmatic signaling involved in short- and long term memory. So far, the gDNA library containing the recognition elements of the proto-oncogene-protein c-Jun in rat *cerebellum* revealed, out of 100 different DNA classes from the library, i.e. sequences belonging to different genes, 33 different c-Jun binding elements. Among those, several novel recognition-element sequences could be identified, e. g. 5'atgattctgccttgctgcm3', 5'aaacagatcatacaagc3' or 5'ggttagctaagagat3'.

In summary, this invention is concerned with a novel technology for the study of *in vivo* interactions between a known biomolecule and its respective recognition element in the genom in conjunction with gene regulation. Our experimental approach enables fishing of DNA protein complexes in large genoms so that protein-DNA interactions for individual transcription factors other biomolecules with affinity to DNA can be studied.

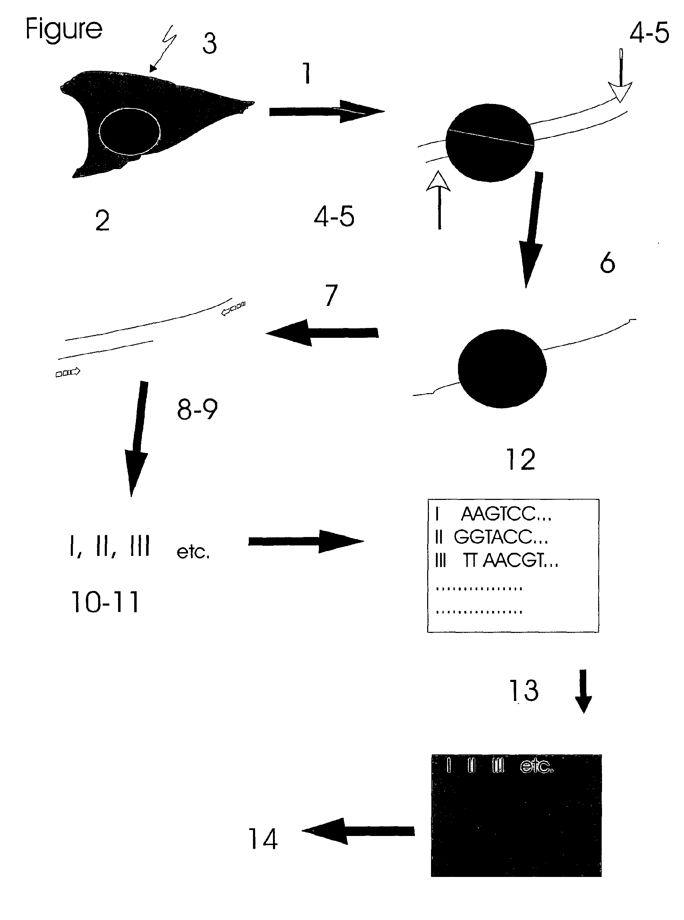

## Claims

1. An universal technique for the study of specific genome-wide protein/DNA-interactions in the context of gene expression, based on the creation of a DNA recognition element library by isolation of specific crosslinked nucleoprotein complexes from cells and the genomic DNA of which is cleaved into restriction fragments containing the DNA-recognition elements with flanking areas, linker ligated, treated with an exonuclease, such as Strandase, treated with a protease, purified and the resulting single-stranded DNA fragments are subjected to primed extension, linker ligation, amplification and cloning before being sequenced and immobilized, for example, on defined positions of a substrate surface for a following hybridization with DNA and or RNA fragments obtained from cells of interest thus allowing the assignment to the corresponding target genes and ascertaining the biomolecular/DNA-recognition elements.
means: the said technique is a new method for the preparation of DNA-recognition element library
means: the said technique allows a genome wide determination of specific protein/molecule/DNA-recognition elements (footprints)
means: the said technique allows the determination of the associated genes in silico.
means: the said technique allows the determination of all DNA sequences if containing both, the recognition elements and parts of the corresponding genes that can be used for the simultaneous expression of the target genes, or are modulated by the complex formation of, for example, transcription factors.
means: the said technique enables simultaneous identification of protein/DNA-recognition elements and expression analysis of target genes and an assessment of its importance for gene expression.
means: the said technique can be used to develop transcription-factors and other DNA-interacting biomolecules-specific libraries
means: the said technique enables identification of genomic targets of drugs, chemicals, toxins, pathogens and environmental factors
means: the said technique can be used for the development of new therapeutic concepts such as gene expression based on drug modulation of protein/DNA-interactions
means: the said technique can be used for elucidating the molecular perturbations and the relative contribution of these events for diseases at the genome level.

2. The technique of claim 1)
can be used in low, medium and high throughput screening systems for the characterization of chemical and biological libraries.

3. The technique of claim 1) can be automated.

4. The technique of claim 1) can be miniaturized.
